# EUROPÄISCHE PATENTANMELDUNG

(11) **EP 4 545 645 A1**
(43) Veröffentlichungstag der Anmeldung: **30.04.2025**
(21) Anmeldenummer: 23206537.5
(22) Anmeldetag: 27.10.2023
(51) Int. Cl.: C12P 7/02, C12N 9/04, C12P 7/24, C12P 7/42

(54) **VERFAHREN ZUR HERSTELLUNG EINER WÄSSERIGEN LÖSUNG ENTHALTEND GLYCOLSÄURE**

(71) Anmelder: Annikki GmbH, 8074 Raaba-Grambach (AT)
(72) Erfinder: Staunig, Nicole, 8074 Raaba-Grambach (AT)
(74) Vertreter: Schwarz & Partner Patentanwälte GmbH

(57) **Zusammenfassung**

Verfahren zur Herstellung einer wässerigen Lösung enthaltend Glycolsäure, indem Ethylenglycol, welches in einer wässerigen Lösung vorliegt, durch Behandeln mit einer ersten NAD(P)-abhängigen Oxidoreduktase und NAD(P)⁺ *in vitro* zu Glycolaldehyd oxidiert und dieser mit einer weiteren NAD(P)-abhängigen Oxidoreduktase und NAD(P)⁺ *in vitro* zu Glycolsäure oxidiert und das durch diese Oxidationen entstandene reduzierte NAD(P)H mittels einer NAD(P)H-Oxidase oder einer Alkoholdehydrogenase wieder oxidiert und damit regeneriert wird. (Figur 1)

## Beschreibung

Die Erfindung betrifft ein Verfahren zur Herstellung einer wässerigen Lösung, welche Glycolsäure enthält.

### Hintergrund der Erfindung

Obwohl Glycolsäure auch natürlich vorkommt, etwa im Rohr- und Rübenzucker, erfolgt ihre Synthese derzeit, wegen der geringen Konzentrationen der Substanz in diesen nachwachsenden Rohstoffen, größtenteils aus fossilen Brennstoffen. Da in der so synthetisierten Glycolsäure oft noch Reste an Formaldehyd vorhanden sind, was vor allem bei deren Verwendung in Kosmetikprodukten (z.B. in Hautpeelings (Sharad, 2013)) als problematisch angesehen wird, besteht ein großer Bedarf nach alternativen Synthesemethoden.

Chemo-enzymatische Methoden zur Glycolsäure-Herstellung umfassen die Umwandlung von aus Formaldehyd und Hydrogencyanid synthetisiertem Glycolonitril zu Glycolsäure mittels Nitrilasen (Panova et al., 2008; Ben-Bassat et al., 2008). Dieses Verfahren kann aufgrund der verwendeten toxischen, energieintensiv hergestellten und generell aus fossilen Quellen stammenden Chemikalien nicht als nachhaltig bezeichnet werden.

Ein chemisches Verfahren zur Erzeugung von Glycolsäure, das einen erneuerbaren Rohstoff als Ausgangsstoff nimmt, ist die Umsetzung von aus Bio-Öl gewonnenem Glyoxal zu Glycolsäure in Gegenwart von Zeolith als Katalysator (Dapsens et al., 2014). Bio-Öl wird durch Pyrolyse von Biomasse gewonnen - ein energiereicher Prozess, bei dem die ursprüngliche Zusammensetzung der Biomasse zerstört wird und das synthetische Potential des Rohstoffes ungenutzt bleibt.

Auch durch die Reaktion von Chloressigsäure (hergestellt durch Chlorierung von Essigsäure) mit Alkalilauge kann Glycolsäure (oder das entsprechende Alkaliglycolat) gebildet werden, allerdings fallen dabei große Mengen an Alkalichloriden als Nebenprodukt an. Darüber hinaus enthält die durch Elektrodialyse gereinigte Glycolsäure noch Spuren von Chloressigsäure sowie Dichloressigsäure (Nebenprodukt der Chlorierung) (EP 0784047 B1).

Glycolsäure kann aber auch durch Oxidation von Ethylenglycol (Ethan-1,2-diol), dem einfachsten zweiwertigen Alkohol, erhalten werden. Die Oxidation kann beispielsweise durch Sauerstoff in Gegenwart des bimetallischen Katalysators AuPt (auf CeO₂ als Trägermaterial) bewerkstelligt werden (Tschirner et al., 2022).

Ethylenglycol wird durch die Hydrolyse von Ethylenoxid (bei 200 °C), welches wiederum durch die Oxidation von Ethen (gewonnen aus fossilen Rohstoffen) erhalten wird, hergestellt. Dabei entstehende

Nebenprodukte (Oligoethylenglycole) müssen destillativ und damit energieintensiv abgetrennt werden. Zur Vermeidung von Nebenprodukten kann beispielsweise der OMEGA-Prozess (Only Mono Ethylene Glycol Advantage) von Shell eingesetzt werden, bei dem Ethylenoxid zuerst mit CO₂ zu Ethylencarbonat umgesetzt wird, welches unter Freisetzung von CO₂ zu Ethylenglycol hydrolysiert wird (mit KI und K₂MoO₄ als Katalysatoren). Andere Verfahren sind die Rh- oder Co-katalysierte Hydrohydroxymethylierung von Formaldehyd oder die Hydroformylierung von Formaldehyd zu Glycolaldehyd, welcher anschließend zu Ethylenglycol reduziert wird (Berger, 2016).

Die genannten Verfahren zur Herstellung von Ethylenglycol können aufgrund der eingesetzten Rohstoffe, Katalysatoren oder Reaktionsbedingungen nicht als nachhaltig oder umweltfreundlich bezeichnet werden.

Es existieren aber auch Alternativen, die auf nachhaltigen Ressourcen basieren. Bestimmte Mikroorganismen können Biomasse zu Ethanol vergären, welcher in Gegenwart von Phosphorsäure oder Aluminiumoxid zu Ethen dehydratisiert werden kann. Dieses kann dann die zuvor beschriebenen Schritte (Oxidation zu Ethylenoxid und darauffolgende Hydrolyse) zum Ethylenglycol durchlaufen (Wong et al., 2023).

Ethylenglycol kann aber auch durch Pyrolyse von Zuckern und der darauffolgenden katalytischen Hydrierung von den durch die Pyrolyse entstandenen C₁-C₃-Oxygenaten wie Glycolaldehyd, Glyoxal, Acetol (Hydroxyaceton) oder Pyruvaldehyd (Methylglyoxal) erhalten werden (US 10077222 B2; Tullo, 2017).

Die Oxidation von Ethylenglycol zu Glycolsäure über das Intermediat Glycolaldehyd kann biotechnologisch bewerkstelligt werden. Unter den Mikroorganismen, die die Oxidation katalysieren, finden sich beispielsweise *Pichia naganishii* AKU 4267 oder *Rhodotorula* sp. 3Pr-126, die 100 g/l Ethylenglycol in 120 h zu 105 g/l bzw. 110 g/l Glycolsäure umsetzen (Kataoka et al., 2001).

Ein weiterer Mikroorganismus, welcher Ethylenglycol zu Glycolsäure oxidieren kann, ist der Hefepilz *Yarrowia lipolytica* IMUFRJ 50682, der bereits als Produzent von diversen Hydrolasen zur Depolymerisation von Poly(ethylenterephthalat) bekannt ist. So konnten *resting cells* von *Y. lipolytica* in einem Bioreaktor 548,6 mM Ethylenglycol (34,1 g/l; 1000 mM Start-Konzentration) innerhalb von 72 h zu 429,5 mM Glycolsäure (32,7 g/l) oxidieren, was einer Ausbeute von 74% (bezogen auf die umgesetzte Substrat-Menge) entspricht (Carniel et al., 2023). Die beteiligten Enzyme werden von Carniel et al. als ähnlich zu den membran-gebundenen Dehydrogenasen von Essigsäurebakterien (*Gluconobacter, Acetobacter*) beschrieben.

Zhang et al. (2016) sowie Hua et al. (2019) beschreiben die Umsetzung von Ethylenglycol zu Glycolsäure durch das industriell häufig eingesetzte Essigsäurebakterium *Gluconobacter oxydans.* So können mit immobilisierten G. oxydans-Zellen in 72 h bis zu 66,9 g/l Glycolsäure (880 mM) aus Ethylenglycol in einem offenen Bioreaktor mit O₂-Begasung hergestellt werden (Hua et al., 2019). Zhang et al. konnten sogar 113,8 g/l Glycolsäure (1,50 M; molare Ausbeute 92,9%) nach 45 h mit einem engineerten *G*. *oxydans-*Stamm erzeugen, bei dem eine membran-gebundene Alkoholdehydrogenase (mADH) überexprimiert wurde (Zhang et al., 2016).

Obwohl Ethylenglycol für *Escherichia coli* kein natürliches Substrat ist, ist *E. coli* in der Lage, Ethylenglycol zu Glycolsäure oxidieren, wenn eine Mutante der Propandiol-Oxidoreduktase (kodiert durch *fucO*) sowie die Glycolaldehyd-Dehydrogenase (kodiert durch *aldA*) überexprimiert werden (Boronat et al., 1983; Pandit et al., 2021).

Der erste Oxidationsschritt (Ethylenglycol zu Glycolaldehyd) kann auch durch eine zytosolische Medium-Chain-Alkoholdehydrogenase (Gox0313) aus *Gluconobacter oxydans* DSM2003 bewerkstelligt werden. Diese kann *in vitro* 500 mM Ethylenglycol zu 484,2 mM Glycolaldehyd in 14 h (30 °C und pH 8,5) umwandeln, wobei die Regeneration des Kofaktors NAD⁺ mit einer NADH-Oxidase (NOX-2) aus *Lactobacillus brevis* ATCC367 erfolgt. Der zweite Oxidationsschritt (Glycolaldehyd zu Glycolsäure) wird von Gox0313 allerdings nicht katalysiert (Zhang et al., 2015).

Eine Alternative zu den bereits angeführten Dehydrogenasen sind Oxidasen. Gao et al. (2014) beschreiben *resting cells* von *Burkholderia* sp. EG13, welche 200 mM Ethylenglycol (12,4 g/l) in 24 h zu Glycolsäure (98,8% Umsatz) oxidieren können. Bei den beteiligten Enzymen handelt es sich hierbei um nicht näher beschriebenen Oxidasen, die einer Substrat-Inhibition ab einer Ethylenglycol-Konzentration von 250 mM sowie einer Produkt-Inhibition ab einer Glycolsäure-Konzentration von 200 mM unterliegen. Um den inhibierenden Effekt der Glycolsäure zu unterdrücken, verwendeten Gao et al. ein Anionentauscherharz zur kontinuierlichen Entfernung von Glycolsäure aus dem Reaktionsgemisch. Somit können im Fed-Batch-Verfahren Produktkonzentrationen von 793 mM (60,3 g/l) erreicht werden.

In einem Übersichtsartikel von Yamada & Isobe (2015) wird ein enzymatisches Verfahren zur Oxidation von Ethylenglycol zu Glycolsäure mit zwei mikrobiellen Oxidasen postuliert. Der erste Schritt wird durch Enzyme aus der Klasse der Alkohol-Oxidasen (EC 1.1.3.13) (z. B. aus *Candida* sp. oder *Pichia pastoris*) oder der Glycerin-Oxidasen (z. B. aus *Aspergillus japonicus*) unter Bildung von Wasserstoffperoxid (H₂O₂) katalysiert. Die abschließende Oxidation kann wiederum durch eine Aldehyd-Oxidase aus *Burkholderia* sp. AlU 129 durchgeführt werden (Yamada & Isobe, 2015; Yamada et al., 2015). Eine von Yamada & Isobe (2015) angekündigte experimentelle Studie zur Optimierung der Reaktionsbedingungen zur Umsetzung von Ethylenglycol zu Glycolsäure mit zwei mikrobiellen Oxidasen ist bis zum jetzigen Zeitpunkt nicht veröffentlicht worden.

Hier setzt nun die vorliegende Erfindung an und setzt sich zum Ziel, die von Yamada & Isobe (2015) sowie von Gao et al. (2014) beschriebenen Verfahren dahingehend zu verbessern, dass eine effiziente Produktion von Glycolsäure unter Vermeidung der Bildung von enzymschädlichem Wasserstoffperoxid als Nebenprodukt sowie der Vermeidung einer Produkt-Inhibierung ermöglicht wird.

### Detaillierte Beschreibung der Erfindung

Die Aufgabe der Erfindung zur Herstellung einer wässerigen Lösung von Glycolsäure wird gelöst, indem Ethylenglycol, welches in einer wässerigen Lösung gelöst vorliegt, durch Behandeln mit einer ersten NAD(P)-abhängigen Oxidoreduktase und NAD(P)⁺ *in vitro* zu Glycolaldehyd oxidiert und dieser mit einer weiteren NAD(P)-abhängigen Oxidoreduktase und NAD(P)⁺ *in vitro* zu Glycolsäure oxidiert und das durch diese Oxidationen entstandene, reduzierte NAD(P)H mittels einer NAD(P)H-Oxidase oder einer Alkoholdehydrogenase wieder oxidiert und damit regeneriert wird.

Im erfindungsgemäßen Verfahren wird die Umsetzung somit nicht fermentativ durchgeführt. Die Enzyme sind also nicht in den Bakterien enthalten, sondern liegen vielmehr als solche in der wässerigen Lösung vor, also *in vitro.*

In einer bevorzugten Ausführungsform der vorliegenden Erfindung ist die erste Oxidoreduktase eine Alkoholdehydrogenase und/oder die weitere Oxidoreduktase eine Aldehyd-Dehydrogenase, insbesondere eine Glycolaldehyd-Dehydrogenase.

Zur Oxidation des Kofaktors NAD(P)H wird bevorzugt eine Alkoholdehydrogenase unter Einsatz eines Ketons oder Aldehyds verwendet, wodurch *in vitro* NAD(P)⁺ und ein sekundärer oder ein primärer Alkohol gebildet werden.

Dadurch kann beispielsweise Aceton, das aus 2-Propanol durch die enzymatische Kofaktorregeneration bei einer enzymatisch katalysierten Reduktion entsteht (siehe EP 2812439 B1 für Beispiele), wieder zu 2-Propanol umgewandelt werden. Dieses kann dann wieder der Reduktionsreaktion zugeführt werden, wodurch ein in sich geschlossener Kreislauf gebildet wird, der die heterogen-katalytische Gasphasenhydrierung von Aceton zu 2-Propanol (Al-Rabiah et al., 2022) obsolet macht.

Zur Oxidation des Kofaktors NAD(P)H wird am besten eine NAD(P)H-Oxidase unter Einsatz von Pressluft, angereichert mit Sauerstoff oder reinem Sauerstoff verwendet, wodurch *in vitro* NAD(P)⁺ und Wasser gebildet werden.

Darüber hinaus ist das Anlegen von Überdruck (Luft oder Sauerstoff) während der Reaktion bevorzugt, um im Falle der Kofaktor-Regeneration durch eine Alkoholdehydrogenase die Verdunstungsverluste des eingesetzten (flüchtigen) Kosubstrats (bevorzugt Aceton) zu begrenzen und im Falle der Kofaktor-Regeneration durch eine NAD(P)H-Oxidase die Löslichkeit des Sauerstoffs im wässrigen Medium zu erhöhen. Überdruck meint in diesem Zusammenhang einen Druck größer als der Umgebungsdruck, bevorzugt mehr als 1,1 bar, noch mehr bevorzugt mehr als 1,2 bar, noch mehr bevorzugt mehr als 1,5 bar, noch mehr bevorzugt mehr als 2 bar, sowie besonders bevorzugt mehr als 5 bar. Die obere Grenze des Überdrucks überschreitet hierbei nicht 20 bar, bevorzugt 10 bar und besonders bevorzugt 5 bar.

Durch den Einsatz von NAD(P)-abhängigen Dehydrogenasen zur Oxidation von Ethylenglycol zu Glycolsäure kann auf die Verwendung von Oxidasen verzichtet werden, die zwei große Nachteile aufweisen: 1. die Bildung von Wasserstoffperoxid als Nebenprodukt der Oxidationen, welches in höheren Konzentrationen schädlich für die eingesetzten Enzyme ist und dessen Entfernung den Zusatz von Katalase erfordert, sowie 2. die Produkt-Inhibierung, wie von Gao et al. (2014) beschrieben. Stattdessen wird auf Enzyme gesetzt, die abhängig vom Kofaktor NAD(P)⁺ sind, welcher durch den Einsatz einer O₂-abhängigen NAD(P)H-Oxidase oder einer Alkoholdehydrogenase mit einem Aldehyd oder Keton (bevorzugt Aceton) als Kosubstrat regeneriert werden kann.

Es hat sich gezeigt, dass es besonders vorteilhaft ist, wenn die Alkoholdehydrogenase sowie die Glycolaldehyd-Dehydrogenase im Reaktionsgemisch als Lysat der entsprechenden, sie bildenden Zellen vorliegen.

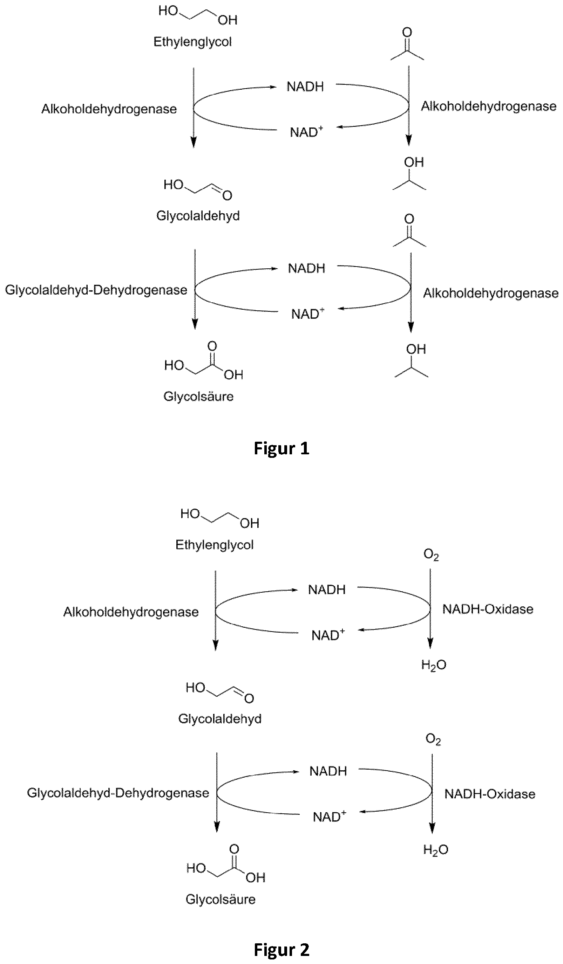

Bevorzugte Varianten der beiden erfindungsgemäßen Verfahren sind in den beiliegenden Figuren 1 und 2 schematisch dargestellt.

In weiteren bevorzugten Ausführungsformen des erfindungsgemäßen Verfahrens beträgt die Konzentration von Ethylenglycol in der wässerigen Lösung 20 - 250 g/l.

Der besonders bevorzugte Temperaturbereich liegt zwischen 20 und 40 °C.

Der besonders bevorzugte pH-Bereich der Reaktion liegt zwischen 6,0 und 9,0.

Um Schaumbildung während des Verfahrens zu unterbinden bzw. zu reduzieren kann ein Antischaummittel zugesetzt werden (z.B. Glanapon 2000; Bussetti und Co., GmbH, Österreich).

In einer weiteren Variante können die Enzyme im Homogenat oder in Pulverform vorliegen, mit einem wasserlöslichen Polymer wie Polyethylenglycol modifiziert, in oder auf einer festen Matrix immobilisiert oder Teil eines Fusionsproteins sein. Zudem können die Enzyme auch in lyophilisierter oder sprühgetrockneter Form vorliegen.

Homogenat steht in diesem Kontext für eine physikalisch und/oder chemisch behandelte Zell-Suspension (z.B. mittels Druckes, Lysozym oder Ultraschall behandelt), wobei die Zellbestandteile aus den Zellen freigesetzt werden. Ein Lysat wird erhalten, wenn die unlöslichen Zellbestandteile des Homogenats beispielsweise durch Filtration oder Zentrifugation entfernt werden (siehe *Produktion der Enzyme & Herstellung der Lysate* für Details).

Bei der zur Oxidation von Ethylenglycol zu Glycolaldehyd eingesetzten Oxidoreduktase handelt es sich bevorzugt um eine Alkoholdehydrogenase (EC 1.1.1.1 oder EC 1.1.1.2).

Die zur Oxidation von Glycolaldehyd zu Glycolsäure verwendete Oxidoreduktase stammt bevorzugt aus der Gruppe EC 1.2.1.21 (Glycolaldehyd-Dehydrogenase).

Die zur Kofaktor-Regeneration eingesetzte NAD(P)H-Oxidase kann aus einer der Gruppen EC 1.6.3.1 (NAD(P)H-Oxidase (H₂O₂-bildend)), EC 1.6.3.2 (NAD(P)H-Oxidase (H₂O-bildend)), EC 1.6.3.3 (NADH-Oxidase (H₂O₂-bildend)) sowie EC 1.6.3.4 (NADH-Oxidase (H₂O-bildend)) stammen, wobei die H₂O-bildenden Klassen besonders bevorzugt sind.

Bei der zur Kofaktor-Regeneration eingesetzten Oxidoreduktase handelt es sich bevorzugt um eine Alkoholdehydrogenase (EC 1.1.1.1 oder EC 1.1.1.2).

Die NAD(P)-abhängige Oxidoreduktase zur Oxidation von Ethylenglycol zu Glycolaldehyd umfasst vorzugsweise eine Aminosäuresequenz, welche ausgewählt ist aus der Gruppe bestehend aus:
i) einer Aminosäuresequenz, die eine Identität zu SEQ ID Nr. 1, 3 oder 5 von mindestens 80% aufweist,
ii) einer Aminosäuresequenz, die durch eine Nukleinsäure kodiert wird, die eine Identität zu SEQ ID Nr. 2, 4 oder 6 von mindestens 80% aufweist, und
iii) einer Aminosäuresequenz, die durch eine Nukleinsäure kodiert wird, die unter stringenten Bedingungen an ein Nukleinsäuremolekül mit der Nukleinsäuresequenz SEQ ID Nr. 2, 4 oder 6 bindet.

SEQ ID Nr. 1: 
MKAAVVEQFKKPLQVKEVEKPKISYGEVLVRIKACGVCHTDLHAAHGDWPVKPKLPLIPGHEGVGVIEEVGPGV
THLKVGDRVGIPWLYSACGHCDYCLSGQETLCERQQNAGYSVDGGYAEYCRAAADYVVKIPDNLSFEEAAPIFC
AGVTTYKALKVTGAKPGEWVAIYGIGGLGHVAVQYAKAMGLNVVAVDLGDEKLELAKQLGADLVVNPKHDD
AAQWIKEKVGGVHATVVTAVSKAAFESAYKSIRRGGACVLVGLPPEEIPIPIFDTVLNGVKIIGSIVGTRKDLQEAL
QFAAEGKVKTIVEVQPLENINDVFDRMLKGQINGRVVLKVD
SEQ ID Nr. 2: 
ATGAAAGCTGCAGTTGTGGAACAATTTAAAAAGCCGTTACAAGTGAAAGAAGTGGAAAAACCTAAGATCT
CATACGGGGAAGTATTAGTGCGCATCAAAGCGTGTGGGGTATGCCATACAGACTTGCATGCCGCACATGG
CGACTGGCCTGTAAAGCCTAAACTGCCTCTCATTCCTGGCCATGAAGGCGTCGGTGTAATTGAAGAAGTAG
GTCCTGGGGTAACACATTTAAAAGTTGGAGATCGCGTAGGTATCCCTTGGCTTTATTCGGCGTGCGGTCAT
TGTGACTATTGCTTAAGCGGACAAGAAACATTATGCGAACGTCAACAAAACGCTGGCTATTCCGTCGATGG
TGGTTATGCTGAATATTGCCGTGCTGCAGCCGATTATGTCGTAAAAATTCCTGATAACTTATCGTTTGAAGA
AGCCGCTCCAATCTTTTGCGCTGGTGTAACAACATATAAAGCGCTCAAAGTAACAGGCGCAAAACCAGGTG
AATGGGTAGCCATTTACGGTATCGGCGGGCTTGGACATGTCGCAGTCCAATACGCAAAGGCGATGGGGTT
AAACGTCGTTGCTGTCGATTTAGGTGATGAAAAACTTGAGCTTGCTAAACAACTTGGTGCAGATCTTGTCG
TCAATCCGAAACATGATGATGCAGCACAATGGATAAAAGAAAAAGTGGGCGGTGTGCATGCGACTGTCGT
CACAGCTGTTTCAAAAGCCGCGTTCGAATCAGCCTACAAATCCATTCGTCGCGGTGGTGCTTGCGTACTCGT
CGGATTACCGCCGGAAGAAATACCTATTCCAATTTTCGATACAGTATTAAATGGAGTAAAAATTATTGGTTC
TATCGTTGGTACGCGCAAAGACTTACAAGAGGCACTTCAATTTGCAGCAGAAGGAAAAGTAAAAACAATT
GTCGAAGTGCAACCGCTTGAAAACATTAACGACGTATTCGATCGTATGTTAAAAGGGCAAATTAACGGCCG
CGTCGTGTTAAAAGTAGATTAA
SEQ ID Nr. 3: 
MSGKMKAAVVHEFGKPLTIEELDIPTIKPTQILVKMIACGVCHTDLHAASGDWPKKPHLPFIPGHEGVGTVVQV
GSEVDWVKEGDVVGVPWLYSACGHCEHCLAGWETLCAKQEETGYSVNGCFAEYVVADPNYIAHLPKGVDPV
KVAPVLCAGLTVYKGLKMTDTRAGNWVAISGVGGLGQMAVQYAVAMGLNVVAVDIDDEKLATAKKLGATYT
VNARNTDPAAFMQEKVGGVHGGLITAVSTKAFSQAMGYVRAGGTLVLNGLPPGDFPISIFDVVMNAITIRGSI
VGTRLDMIEALSFFAEGKVTSVTTTDRIDNINAIFDALKNGRVEGRVVLDFRN
SEQ ID Nr. 4: 
ATGTCCGGAAAAATGAAAGCCGCTGTGGTTCATGAATTTGGCAAACCACTGACCATTGAAGAACTGGACAT
TCCCACCATTAAACCCACCCAGATTTTGGTTAAAATGATTGCCTGCGGCGTGTGCCATACAGATTTGCATGC
TGCCAGCGGGGATTGGCCCAAAAAACCACATCTGCCGTTTATTCCCGGCCACGAAGGCGTTGGCACGGTTG
 
TGCAGGTTGGCAGCGAAGTAGACTGGGTAAAGGAAGGCGACGTTGTTGGCGTGCCTTGGCTCTATTCTGC
CTGCGGCCATTGCGAGCATTGTCTGGCTGGGTGGGAAACCCTTTGTGCGAAGCAAGAAGAAACCGGCTAT
TCCGTAAACGGCTGCTTTGCCGAATACGTGGTAGCAGACCCTAACTACATTGCTCATCTTCCCAAAGGCGTA
GACCCTGTAAAAGTTGCCCCCGTACTGTGCGCAGGCCTGACCGTTTATAAGGGCCTGAAAATGACAGACAC
GCGGGCAGGGAACTGGGTTGCCATTTCCGGCGTTGGGGGGTTAGGCCAGATGGCTGTGCAGTACGCCGT
AGCAATGGGGCTGAATGTTGTGGCGGTAGATATTGATGATGAAAAACTGGCAACCGCCAAAAAGCTTGGT
GCAACGTACACTGTTAACGCCAGAAATACAGATCCGGCAGCATTTATGCAGGAAAAAGTTGGTGGCGTAC
ACGGTGGATTGATCACCGCTGTTTCCACCAAAGCATTTTCTCAGGCTATGGGTTATGTGCGGGCAGGGGGC
ACTTTGGTGCTAAATGGGCTGCCGCCGGGTGATTTCCCAATTTCTATCTTTGACGTGGTCATGAATGCCATT
ACCATTCGCGGCTCCATAGTAGGCACACGGCTGGACATGATTGAAGCTCTTTCCTTCTTTGCGGAAGGGAA
GGTAACATCTGTCACCACAACGGATCGGATTGATAACATCAACGCAATTTTTGATGCGCTCAAGAACGGTC
GGGTGGAAGGCCGCGTGGTTCTGGACTTCCGCAACTGA
SEQ ID Nr. 5: 
MNKTMKAAVARAFGKPLEIEEVEVPRPRAGELLVKIEACGVCHTDLHAVEGDWPVKPNPPFIPGHEGVGHVV
AVGEGVTHVKEGDRVGIPWLYSACGHCEHCLGGWETLCEQQQNAGYSVNGGFAEYALAAADYVGLLPKNVG
FVDIAPVLCAGVTVYKGLKMTDTRPGNWVVVSGIGGLGHMAVQYARAMGLNVAAVDIDDDKLDFAKRLGAE
VVVNAKATDPAAYLKKEIGGAHGALITAVSPKAFEQALGMVRRGGTVALNGLPPGDFPLSIFDMVLNGVTVRG
SIVGSRLDLQESLQFAEEGKVRATVATEKLENINSVFDRMRRGQIEGRIVLDMAA
SEQ ID Nr. 6:

ATGAACAAGACCATGAAGGCCGCGGTGGCCCGCGCGTTCGGCAAACCCCTGGAAATCGAAGAAGTCGAG
GTGCCGCGCCCGCGCGCGGGCGAACTGTTGGTGAAGATCGAGGCCTGCGGCGTCTGCCACACCGATCTGC
ACGCGGTCGAGGGCGACTGGCCGGTCAAGCCCAATCCCCCTTTCATCCCGGGCCACGAAGGCGTGGGCCA
CGTGGTGGCGGTGGGCGAAGGCGTGACGCACGTCAAGGAAGGCGACCGCGTCGGCATCCCCTGGCTGTA
TTCCGCCTGTGGCCATTGCGAGCATTGCCTGGGCGGCTGGGAGACGCTGTGCAACAGCAGCAGAACGCC
GGCTACTCCGTCAACGGCGGCTTCGCCGAGTACGCGCTGGCCGCGGCCGACTACGTGGGCCTGCTGCCGA
AGAATGTCGGCTTCGTCGACATCGCCCCGGTACTGTGCGCCGGCGTCACGGTCTACAAAGGCCTGAAGAT
GACGGACACACGGCCAGGCAACTGGGTCGTGGTCTCCGGCATCGGCGGCCTGGGCCACATGGCGGTGCA
GTATGCGCGCGCCATGGGCTTGAACGTGGCGGCCGTGGACATCGACGACGACAAACTGGATTTCGCCAAG
CGTCTCGGCGCCGAGGTGGTCGTGAATGCCAAGGCCACCGATCCAGCGGCTTACCTGAAGAAGGAAATCG
GCGGCGCCCACGGCGCGCTGATCACGGCGGTTTCGCCCAAGGCCTTCGAGCAGGCGCTGGGCATGGTGC
GTCGCGGCGGCACGGTGGCGCTCAACGGCCTGCCGCCGGGCGACTTCCCGCTGTCGATCTTCGACATGGT
GCTCAACGGCGTGACGGTGCGCGGTTCGATCGTGGGTTCGCGCCTGGACTTGCAGGAATCGCTGCAGTTC

GCCGAGGAAGGCAAGGTGCGCGCGACCGTGGCGACCGAGAAGCTGGAGAACATCAACAGCGTGTTCGAC
CGCATGCGCCGCGGCCAGATCGAAGGCCGCATCGTGCTGGACATGGCGGCGTAA

Die NAD(P)-abhängige Oxidoreduktase zur Oxidation von Ethylenglycol zu Glycolaldehyd umfasst oder besteht vorzugsweise aus einer Aminosäuresequenz, die eine Identität zu SEQ ID Nr. 1, 3 oder 5 von mindestens 80%, noch mehr bevorzugt von 85%, noch mehr bevorzugt von 90%, noch mehr bevorzugt von 95%, noch mehr bevorzugt von 98%, noch mehr bevorzugt von 99%, insbesondere von 100%, aufweist. Besonders bevorzugt umfasst die erfindungsgemäße NAD(P)-abhängige Oxidoreduktase zur Oxidation von Ethylenglycol zu Glycolaldehyd die Aminosäuresequenz SEQ ID Nr. 1, 3 oder 5 oder besteht aus dieser.

Alternativ umfasst oder besteht die NAD(P)-abhängige Oxidoreduktase zur Oxidation von Ethylenglycol zu Glycolaldehyd vorzugsweise aus einer Aminosäuresequenz, die durch eine Nukleinsäure kodiert wird, die eine Identität zu SEQ ID Nr. 2, 4 oder 6 von mindestens 80%, noch mehr bevorzugt von 85%, noch mehr bevorzugt von 90%, noch mehr bevorzugt von 95%, noch mehr bevorzugt von 98%, noch mehr bevorzugt von 99%, insbesondere von 100%, aufweist. Besonders bevorzugt umfasst die Nukleinsäure, welche die erfindungsgemäße NAD(P)-abhängige Oxidoreduktase zur Oxidation von Ethylenglycol zu Glycolaldehyd kodiert, die Nukleinsäuresequenz SEQ ID Nr. 2, 4 oder 6 oder besteht aus dieser.

Der Begriff "Identität", wie hier verwendet, bezieht sich auf den Prozentsatz der identischen Nukleotid- bzw. Aminosäureübereinstimmungen zwischen mindestens zwei, unter Verwendung eines standardisierten Algorithmus miteinander ausgerichteten Nukleotid- bzw. Aminosäuresequenzen ("Alignment"). Ein solcher Algorithmus kann, in einer standardisierten und reproduzierbaren Weise, Lücken ("Gap") in die verglichenen Sequenzen einfügen, um das Alignment zwischen zwei Sequenzen zu optimieren, und somit einen aussagekräftigeren Vergleich der beiden Sequenzen erreichen.

Die prozentuale Identität zwischen Sequenzen kann unter Verwendung von ein oder mehreren Computeralgorithmen oder im Stand der Technik bekannten oder hierin beschriebenen Programmen bestimmt werden. Erfindungsgemäß wird zur Bestimmung der Identität das durch National Center for Biotechnology Information (NCBI) bereitgestellte Basic Local Alignment Search Tool (BLAST) (Altschul et al., 1990), verwendet. Die BLAST-Software-Reihe enthält verschiedene Programme, einschließlich ein als "BLAST 2 Sequences" genanntes Werkzeug, das für den direkten paarweisen Vergleich von zwei Nukleotid- bzw. Aminosäuresequenzen verwendet wird. "BLAST 2 Sequences" kann auch über die NCBI World Wide Web-Seite im Internet interaktiv abgerufen und verwendet werden. Das blastn-Programm (für Nukleotidsequenzen) verwendet als Vorgaben eine Wortlänge (W) von 28, eine Erwartung (E) von 0,05, M = 1, N = -2 und einen Vergleich beider Stränge. Für Aminosäuresequenzen verwendet das blastp-Programm als Vorgaben eine Wortlänge von 3 und eine Erwartung (E) von 0,05 und die BLOSUM62-Scoring-Matrix (Henikoff & Henikoff, 1989), Alignments (B) von 50, Erwartung (E) von 0,05, M = 1, N = - 2.

Alternativ umfasst die NAD(P)-abhängige Oxidoreduktase zur Oxidation von Ethylenglycol zu Glycolaldehyd vorzugsweise eine Aminosäuresequenz oder besteht aus dieser, die durch eine Nukleinsäure kodiert wird, die unter stringenten Bedingungen an ein Nukleinsäuremolekül mit der Nukleinsäuresequenz SEQ ID Nr. 2, 4 oder 6 bindet. Wie hierin verwendet, beziehen sich die stringenten Bedingungen auf Bedingungen, unter denen sogenannte spezifische Hybride, jedoch keine unspezifischen Hybride gebildet werden.

Beispielsweise kann die Hybridisierung durch herkömmlich bekannte Verfahren durchgeführt werden, wie die von J. Sambrook et al. in Molecular Cloning, A Laboratory Manual, 2nd Ed., Cold Spring Harbor Laboratory (1989) beschriebenen. Unter stringenter Bedingung ist eine Bedingung gemeint, bei der das Waschen bei 65 °C und einer Salzkonzentration von 0,1x bis 2x SSC durchgeführt wird (wobei unter 1xSSC eine Mischung aus 0,15 M Natriumchlorid/0,015 M Natriumcitrat zu verstehen ist).

Die erfindungsgemäß eingesetzten Enzyme zur Oxidation von Ethylenglycol zu Glycolaldehyd sind vorzugsweise NAD(P)-abhängige Alkoholdehydrogenasen. Es hat sich überraschenderweise gezeigt, dass diese Enzyme in der Lage sind, Ethylenglycol in Anwesenheit des Kofaktors NAD(P)⁺ zu Glycolaldehyd umzusetzen. Dementsprechend betrifft ein weiterer Aspekt der vorliegenden Erfindung die Verwendung dieser Enzyme zur Oxidation von Ethylenglycol zu Glycolaldehyd in Anwesenheit des Kofaktors NAD(P)⁺.

Die NAD(P)-abhängige Alkoholdehydrogenase zur Oxidation von Ethylenglycol zu Glycolaldehyd umfasst vorzugsweise eine Aminosäuresequenz, welche ausgewählt ist aus der Gruppe bestehend aus:
i) einer Aminosäuresequenz, die eine Identität zu SEQ ID Nr. 1, 3 oder 5 von mindestens 80% aufweist,
ii) einer Aminosäuresequenz, die durch eine Nukleinsäure kodiert wird, die eine Identität zu SEQ ID Nr. 2, 4 oder 6 von mindestens 80% aufweist, und
iii) einer Aminosäuresequenz, die durch eine Nukleinsäure kodiert wird, die unter stringenten Bedingungen an ein Nukleinsäuremolekül mit der Nukleinsäuresequenz SEQ ID Nr. 2, 4 oder 6 bindet.

Gemäß einer besonders bevorzugten Ausführungsform der vorliegenden Erfindung umfasst die zur Kofaktor-Regeneration eingesetzte NAD(P)H-Oxidase eine Aminosäuresequenz, welche ausgewählt ist aus der Gruppe bestehend aus:
i) einer Aminosäuresequenz, die eine Identität zu SEQ ID Nr. 7 oder SEQ ID Nr. 9 von mindestens 80% aufweist,
ii) einer Aminosäuresequenz, die durch eine Nukleinsäure kodiert wird, die eine Identität zu SEQ ID Nr. 8 oder SEQ ID Nr. 10 von mindestens 80% aufweist, und
iii) einer Aminosäuresequenz, die durch eine Nukleinsäure kodiert wird, die unter stringenten Bedingungen an ein Nukleinsäuremolekül mit der Nukleinsäuresequenz SEQ ID Nr. 8 oder SEQ ID Nr. 10 bindet.

SEQ ID Nr. 7: 
MKVVVVGCTHAGTAAVKTILNEHPDASVSVYERNDNVSFLSCGIALYVGGVVKDPAGLFYSSPEELASMGAKIN
MEHNVKNIDNENKVVVIENLKTGETFEESYDKLVMTTGSWPIIPPIDGINSENILLCKNYNQANEIIKESKNAKKIV
IVGGGYIGIELVEAFAESGKQVTLVDGLDRILNKYLDAEFTSVLEHDLQERGVTLALNQTVEKFVANESGAVTAVK
TPVGEYEADLVILCVGFKPNTDLLKDKVEMLPNGAIVVDEYMRTSDEAIFAAGDSCAVHYNPTGGSAYIPLATN
AVRMGALVGKNIVSPTVKYRGTQATSGLYLFGFNIGSTGLTENSAPHFGVEVRSVVVEDNYRPEFMPTTEKVT
MKLVYEVGTNRIVGGQIMSKYDVTQSANTLSLCVQNKMTIEDLAYVDFFFQPHFDRPWNYLNILAQAAVEQER
KLAK
SEQ ID Nr. 8: 
ATGAAAGTAGTAGTAGTAGGCTGTACACATGCAGGAACAGCGGCAGTTAAGACGATTTTAAATGAACATC
CAGATGCATCAGTATCAGTATATGAGCGTAATGACAATGTCTCATTTCTATCTTGTGGGATTGCGTTGTATG
TTGGTGGAGTTGTGAAAGATCCTGCAGGTTTGTTTTATTCAAGTCCAGAAGAACTTGCATCAATGGGCGCG
AAAATTAACATGGAACACAATGTGAAAAATATAGATAATGAGAATAAGGTCGTAGTAATTGAGAATTTAAA
AACAGGCGAAACATTTGAAGAAAGCTATGATAAGTTGGTAATGACAACTGGATCATGGCCAATTATTCCTC
CAATTGATGGAATCAATAGTGAAAATATTCTTTTGTGTAAAAACTATAACCAAGCAAATGAAATTATTAAAG
AATCAAAAAATGCTAAAAAGATTGTCATTGTTGGTGGTGGCTATATTGGAATTGAATTAGTTGAGGCATTT
GCAGAATCTGGCAAGCAAGTGACGCTAGTTGATGGATTAGATCGTATTTTAAACAAATATTTAGATGCTGA
ATTCACTTCTGTTTTAGAGCATGATTTACAAGAAAGAGGCGTTACGCTAGCTTTAAACCAAACCGTCGAGAA
ATTTGTTGCCAATGAATCAGGTGCTGTGACAGCTGTGAAAACACCAGTTGGAGAATATGAGGCTGATTTAG
TTATTTTATGTGTTGGATTTAAACCAAATACTGATTTGTTGAAGGATAAAGTAGAGATGTTGCCAAATGGTG
CCATCGTAGTGGATGAATATATGAGAACAAGCGATGAAGCGATTTTTGCTGCTGGCGATAGTTGCGCGGTT
 
CATTATAATCCAACTGGAGGCTCTGCGTATATTCCGTTAGCTACAAATGCAGTTAGAATGGGAGCTTTAGTT
GGGAAAAATATTGTTTCTCCAACAGTTAAATATCGTGGCACGCAAGCAACTTCTGGTTTATATTTATTTGGT
TTTAATATAGGTTCAACCGGATTGACTGAAAATAGCGCTCCTCATTTTGGCGTAGAGGTTCGTTCAGTAGTT
GTAGAAGATAATTATCGTCCAGAGTTTATGCCGACAACAGAGAAAGTAACGATGAAATTAGTTTATGAAGT
AGGAACGAATCGGATTGTTGGAGGTCAAATCATGTCAAAATATGATGTGACACAATCTGCCAATACGTTAT
CTTTATGTGTTCAAAATAAAATGACGATTGAGGATTTGGCTTATGTAGATTTCTTCTTCCAACCTCACTTTGA
TCGTCCTTGGAACTATTTAAATATTTTAGCGCAAGCAGCTGTTGAGCAAGAGCGTAAACTAGCAAAATAA
SEQ ID Nr. 9: 
MSKIVIVGANHAGTAAINTILDNYGSENEVVVFDQNSNISFLGCGMALWIGKQISGPQGLFYADKESLEAKGAKI
YMESPVTAIDYDAKRVTALVNGQEHVESYEKLILATGSTPILPPIKGAAIKEGSRDFEATLKNLQFVKLYQNAEDVI
NKLQDKSQNLNRIAVVGAGYIGVELAEAFKRLGKEVILIDVVDTCLAGYYDQDLSEMMRQNLEDHGIELAFGET
VKAIEGDGKVERIVTDKASHDVDMVILAVGFRPNTALGNAKLKTFRNGAFLVDKKQETSIPDVYAIGDCATVYD
NAINDTNYIALASNALRSGIVAGHNAAGHKLESLGVQGSNGISIFGLNMVSTGLTQEKAKRFGYNPEVTAFTDF
QKASFIEHDNYPVTLKIVYDKDSRLVLGAQMASKEDMSMGIHMFSLAIQEKVTIERLALLDYFFLPHFNQPYNY
MTKAALKAK
SEQ ID Nr. 10:

ATGAGCAAAATTGTTATCGTGGGTGCAAATCATGCAGGCACCGCAGCAATTAATACCATTCTGGATAATTA
TGGCAGCGAAAATGAAGTGGTTGTGTTTGATCAGAATAGCAACATTAGCTTTCTGGGTTGTGGTATGGCAC
TGTGGATTGGTAAACAAATTAGCGGTCCGCAGGGTCTGTTTTATGCAGATAAAGAAAGCCTGGAAGCAAA
AGGTGCCAAAATCTATATGGAAAGTCCGGTTACCGCCATTGATTATGATGCAAAACGTGTTACCGCACTGG
TTAATGGTCAAGAACATGTTGAAAGCTACGAGAAACTGATTCTGGCAACCGGTAGCACCCCGATTCTGCCT
CCGATTAAAGGTGCAGCCATTAAAGAAGGTAGTCGCGATTTTGAAGCAACCCTGAAAAATCTGCAGTTCGT
GAAACTGTATCAGAATGCCGAAGATGTGATTAACAAACTGCAGGATAAAAGCCAGAATCTGAATCGTATT
GCAGTTGTTGGTGCAGGTTATATTGGTGTTGAACTGGCAGAAGCATTTAAACGTCTGGGTAAAGAAGTGA
TTCTGATTGACGTTGTTGATACCTGTCTGGCAGGTTATTATGATCAGGATCTGAGCGAAATGATGCGTCAG
AATCTGGAAGATCATGGTATCGAACTGGCATTTGGTGAAACCGTTAAAGCAATTGAAGGTGATGGTAAAG
TGGAACGTATTGTTACCGATAAAGCAAGCCATGATGTGGATATGGTTATTCTGGCAGTTGGTTTTCGTCCG
AATACAGCACTGGGTAATGCAAAACTGAAAACCTTTCGTAATGGTGCCTTTCTGGTGGATAAAAAACAAGA
AACCAGCATCCCGGATGTTTATGCAATTGGTGATTGTGCAACCGTGTATGATAATGCCATTAACGACACCA
ACTATATTGCACTGGCAAGCAATGCACTGCGTAGCGGTATTGTTGCAGGTCATAATGCAGCCGGTCATAAA
CTGGAAAGTCTGGGTGTTCAGGGTAGCAATGGTATTTCAATTTTTGGCCTGAATATGGTTAGCACCGGTCT
GACCCAAGAAAAAGCCAAACGTTTTGGTTATAATCCGGAAGTTACCGCCTTTACCGATTTTCAGAAAGCCA

GCTTTATCGAGCATGATAACTATCCGGTTACGCTGAAAATTGTGTATGACAAAGATAGCCGTCTGGTTCTG
GGTGCACAGATGGCCAGCAAAGAAGATATGAGCATGGGTATTCACATGTTTAGCCTGGCCATTCAAGAGA
AAGTTACCATTGAACGTCTGGCCCTGCTGGATTATTTCTTTCTGCCGCATTTTAATCAGCCGTACAACTATAT
GACCAAAGCAGCACTGAAAGCCAAATAA

Die NAD(P)H-Oxidase, die ausgewählt ist aus der Gruppe bestehend aus einer Aminosäuresequenz, die eine Identität zu SEQ ID Nr. 7 von mindestens 80% aufweist, einer Aminosäuresequenz, die durch eine Nukleinsäure kodiert wird, die eine Identität zu SEQ ID Nr. 8 von mindestens 80% aufweist, und einer Aminosäuresequenz, die durch eine Nukleinsäure kodiert wird, die unter stringenten Bedingungen an ein Nukleinsäuremolekül mit der Nukleinsäuresequenz SEQ ID Nr. 8 bindet, eignet sich besonders gut für das erfindungsgemäße Verfahren, da diese eine im Vergleich zu anderen NAD(P)H-Oxidasen aus dem Stand der Technik signifikant höhere Stabilität aufweist.

Alternativ oder zusätzlich zu den zuvor genannten NAD(P)H Oxidasen können auch Varianten der NAD(P)H-Oxidase mit der Aminosäuresequenz SEQ ID Nr. 7 eingesetzt werden. Besonders bevorzugt sind dabei Varianten mit einer der Aminosäuresequenzen SEQ ID Nr. 11, SEQ ID Nr. 13, SEQ ID Nr. 15 oder SEQ ID Nr. 17, welche vorzugsweise durch Nukleinsäuremoleküle kodiert werden, welche die Nukleinsäuresequenzen SEQ ID Nr. 12, SEQ ID Nr. 14, SEQ ID Nr. 16 bzw. SEQ ID Nr. 18 umfassen.
SEQ ID Nr. 11: 
MKVVVVGCTHAGTAAVKTILNEHPDASVSVYERNDNVSFLSCGIALYVGGVVKDPAGLFYSSPEELASMGAKIN
MEHNVKNIDNENKVVVIENLKTGETFEESYDKLVMTTGSWPIIPPIDGINSENILLCKNYNQANEIIKESKNAKKIV
IVGGGYIGIELVEAFAESGKQVTLVARSDRILRKYLDAEFTSVLEHDLQERGVTLALNQTVEKFVANESGAVTAVK
TPVGEYEADLVILCVGFKPNTDLLKDKVEMLPNGAIVVDEYMRTSDEAIFAAGDSCAVHYNPTGGSAYIPLATN
AVRMGALVGKNIVSPTVKYRGTQATSGLYLFGFNIGSTGLTENSAPHFGVEVRSVVVEDNYRPEFMPTTEKVT
MKLVYEVGTNRIVGGQIMSKYDVTQSANTLSLCVQNKMTIEDLAYVDFFFQPHFDRPWNYLNILAQAAVEQER
KLAK
SEQ ID Nr. 12: 
ATGAAAGTAGTAGTAGTAGGCTGTACACATGCAGGAACAGCGGCAGTTAAGACGATTTTAAATGAACATC
CAGATGCATCAGTATCAGTATATGAGCGTAATGACAATGTCTCATTTCTATCTTGTGGGATTGCGTTGTATG
TTGGTGGAGTTGTGAAAGATCCTGCAGGTTTGTTTTATTCAAGTCCAGAAGAACTTGCATCAATGGGCGCG
AAAATTAACATGGAACACAATGTGAAAAATATAGATAATGAGAATAAGGTCGTAGTAATTGAGAATTTAAA
AACAGGCGAAACATTTGAAGAAAGCTATGATAAGTTGGTAATGACAACTGGATCATGGCCAATTATTCCTC
CAATTGATGGAATCAATAGTGAAAATATTCTTTTGTGTAAAAACTATAACCAAGCAAATGAAATTATTAAAG
 
AATCAAAAAATGCTAAAAAGATTGTCATTGTTGGTGGTGGCTATATTGGAATTGAATTAGTTGAGGCATTT
GCAGAATCTGGCAAGCAAGTGACGCTAGTTGCGCGTAGCGATCGTATTTTACGTAAATATTTAGATGCTGA
ATTCACTTCTGTTTTAGAGCATGATTTACAAGAAAGAGGCGTTACGCTAGCTTTAAACCAAACCGTCGAGAA
ATTTGTTGCCAATGAATCAGGTGCTGTGACAGCTGTGAAAACACCAGTTGGAGAATATGAGGCTGATTTAG
TTATTTTATGTGTTGGATTTAAACCAAATACTGATTTGTTGAAGGATAAAGTAGAGATGTTGCCAAATGGTG
CCATCGTAGTGGATGAATATATGAGAACAAGCGATGAAGCGATTTTTGCTGCTGGCGATAGTTGCGCGGTT
CATTATAATCCAACTGGAGGCTCTGCGTATATTCCGTTAGCTACAAATGCAGTTAGAATGGGAGCTTTAGTT
GGGAAAAATATTGTTTCTCCAACAGTTAAATATCGTGGCACGCAAGCAACTTCTGGTTTATATTTATTTGGT
TTTAATATAGGTTCAACCGGATTGACTGAAAATAGCGCTCCTCATTTTGGCGTAGAGGTTCGTTCAGTAGTT
GTAGAAGATAATTATCGTCCAGAGTTTATGCCGACAACAGAGAAAGTAACGATGAAATTAGTTTATGAAGT
AGGAACGAATCGGATTGTTGGAGGTCAAATCATGTCAAAATATGATGTGACACAATCTGCCAATACGTTAT
CTTTATGTGTTCAAAATAAAATGACGATTGAGGATTTGGCTTATGTAGATTTCTTCTTCCAACCTCACTTTGA
TCGTCCTTGGAACTATTTAAATATTTTAGCGCAAGCAGCTGTTGAGCAAGAGCGTAAACTAGCAAAATAA
SEQ ID Nr. 13: 
MKVVVVGCTHAGTAAVKTILNEHPDASVSVYERNDNVSFLSCGIALYVGGVVKDPAGLFYSSPEELASMGAKIN
MEHNVKNIDNENKVVVIENLKTGETFEESYDKLVMTTGSWPIIPPIDGINSENILLCKNYNQANEIIKESKNAKKIV
IVGGGYIAIELVEAFAESGKQVTLVARSDRILRKYLDAEFTSVLEHDLQERGVTLALNQTVEKFVANESGAVTAVK
TPVGEYEADLVILCVGFKPNTDLLKDKVEMLPNGAIVVDEYMRTSDEAIFAAGDSCAVHYNPTGGSAYIPLATN
AVRMGALVGKNIVSPTVKYRGTQATSGLYLFGFNIGSTGLTENSAPHFGVEVRSVVVEDNYRPEFMPTTEKVT
MKLVYEVGTNRIVGGQIMSKYDVTQSANTLSLCVQNKMTIEDLAYVDFFFQPHFDRPWNYLNILAQAAVEQER
KLAK
SEQ ID Nr. 14: 
ATGAAAGTAGTAGTAGTAGGCTGTACACATGCAGGAACAGCGGCAGTTAAGACGATTTTAAATGAACATC
CAGATGCATCAGTATCAGTATATGAGCGTAATGACAATGTCTCATTTCTATCTTGTGGGATTGCGTTGTATG
TTGGTGGAGTTGTGAAAGATCCTGCAGGTTTGTTTTATTCAAGTCCAGAAGAACTTGCATCAATGGGCGCG
AAAATTAACATGGAACACAATGTGAAAAATATAGATAATGAGAATAAGGTCGTAGTAATTGAGAATTTAAA
AACAGGCGAAACATTTGAAGAAAGCTATGATAAGTTGGTAATGACAACTGGATCATGGCCAATTATTCCTC
CAATTGATGGAATCAATAGTGAAAATATTCTTTTGTGTAAAAACTATAACCAAGCAAATGAAATTATTAAAG
AATCAAAAAATGCTAAAAAGATTGTCATTGTTGGTGGTGGCTATATTGCGATTGAATTAGTTGAGGCATTT
GCAGAATCTGGCAAGCAAGTGACGCTAGTTGCGCGTAGCGATCGTATTTTACGTAAATATTTAGATGCTGA
ATTCACTTCTGTTTTAGAGCATGATTTACAAGAAAGAGGCGTTACGCTAGCTTTAAACCAAACCGTCGAGAA
ATTTGTTGCCAATGAATCAGGTGCTGTGACAGCTGTGAAAACACCAGTTGGAGAATATGAGGCTGATTTAG
 
TTATTTTATGTGTTGGATTTAAACCAAATACTGATTTGTTGAAGGATAAAGTAGAGATGTTGCCAAATGGTG
CCATCGTAGTGGATGAATATATGAGAACAAGCGATGAAGCGATTTTTGCTGCTGGCGATAGTTGCGCGGTT
CATTATAATCCAACTGGAGGCTCTGCGTATATTCCGTTAGCTACAAATGCAGTTAGAATGGGAGCTTTAGTT
GGGAAAAATATTGTTTCTCCAACAGTTAAATATCGTGGCACGCAAGCAACTTCTGGTTTATATTTATTTGGT
TTTAATATAGGTTCAACCGGATTGACTGAAAATAGCGCTCCTCATTTTGGCGTAGAGGTTCGTTCAGTAGTT
GTAGAAGATAATTATCGTCCAGAGTTTATGCCGACAACAGAGAAAGTAACGATGAAATTAGTTTATGAAGT
AGGAACGAATCGGATTGTTGGAGGTCAAATCATGTCAAAATATGATGTGACACAATCTGCCAATACGTTAT
CTTTATGTGTTCAAAATAAAATGACGATTGAGGATTTGGCTTATGTAGATTTCTTCTTCCAACCTCACTTTGA
TCGTCCTTGGAACTATTTAAATATTTTAGCGCAAGCAGCTGTTGAGCAAGAGCGTAAACTAGCAAAATAA
SEQ ID Nr. 15: 
MKVVVVGCTHAGTAAVKTILNEHPDASVSVYERNDNVSFLSCGIALYVGGVVKDPAGLFYSSPEELASMGAKIN
MEHNVKNIDNENKVVVIENLKTGETFEESYDKLVMTTGSWPIIPPIDGINSENILLCKNYNQANEIIKESKNAKKIV
IVGGGYIGIELVEAFAESNKQVTLVARSDRILRKYLDAEFTSVLEHDLQERGVTLALNQTVEKFVANESGAVTAVK
TPVGEYEADLVILCVGFKPNTDLLKDKVEMLPNGAIVVDEYMRTSDEAIFAAGDSCAVHYNPTGGSAYIPLATN
AVRMGALVGKNIVSPTVKYRGTQATSGLYLFGFNIGSTGLTENSAPHFGVEVRSVVVEDNYRPEFMPTTEKVT
MKLVYEVGTNRIVGGQIMSKYDVTQSANTLSLCVQNKMTIEDLAYVDFFFQPHFDRPWNYLNILAQAAVEQER
KLAK
SEQ ID Nr. 16: 
ATGAAAGTAGTAGTAGTAGGCTGTACACATGCAGGAACAGCGGCAGTTAAGACGATTTTAAATGAACATC
CAGATGCATCAGTATCAGTATATGAGCGTAATGACAATGTCTCATTTCTATCTTGTGGGATTGCGTTGTATG
TTGGTGGAGTTGTGAAAGATCCTGCAGGTTTGTTTTATTCAAGTCCAGAAGAACTTGCATCAATGGGCGCG
AAAATTAACATGGAACACAATGTGAAAAATATAGATAATGAGAATAAGGTCGTAGTAATTGAGAATTTAAA
AACAGGCGAAACATTTGAAGAAAGCTATGATAAGTTGGTAATGACAACTGGATCATGGCCAATTATTCCTC
CAATTGATGGAATCAATAGTGAAAATATTCTTTTGTGTAAAAACTATAACCAAGCAAATGAAATTATTAAAG
AATCAAAAAATGCTAAAAAGATTGTCATTGTTGGTGGTGGCTATATTGGAATTGAATTAGTTGAGGCATTT
GCAGAATCTAACAAGCAAGTGACGCTAGTTGCGCGTAGCGATCGTATTTTACGTAAATATTTAGATGCTGA
ATTCACTTCTGTTTTAGAGCATGATTTACAAGAAAGAGGCGTTACGCTAGCTTTAAACCAAACCGTCGAGAA
ATTTGTTGCCAATGAATCAGGTGCTGTGACAGCTGTGAAAACACCAGTTGGAGAATATGAGGCTGATTTAG
TTATTTTATGTGTTGGATTTAAACCAAATACTGATTTGTTGAAGGATAAAGTAGAGATGTTGCCAAATGGTG
CCATCGTAGTGGATGAATATATGAGAACAAGCGATGAAGCGATTTTTGCTGCTGGCGATAGTTGCGCGGTT
CATTATAATCCAACTGGAGGCTCTGCGTATATTCCGTTAGCTACAAATGCAGTTAGAATGGGAGCTTTAGTT
GGGAAAAATATTGTTTCTCCAACAGTTAAATATCGTGGCACGCAAGCAACTTCTGGTTTATATTTATTTGGT
 
TTTAATATAGGTTCAACCGGATTGACTGAAAATAGCGCTCCTCATTTTGGCGTAGAGGTTCGTTCAGTAGTT
GTAGAAGATAATTATCGTCCAGAGTTTATGCCGACAACAGAGAAAGTAACGATGAAATTAGTTTATGAAGT
AGGAACGAATCGGATTGTTGGAGGTCAAATCATGTCAAAATATGATGTGACACAATCTGCCAATACGTTAT
CTTTATGTGTTCAAAATAAAATGACGATTGAGGATTTGGCTTATGTAGATTTCTTCTTCCAACCTCACTTTGA
TCGTCCTTGGAACTATTTAAATATTTTAGCGCAAGCAGCTGTTGAGCAAGAGCGTAAACTAGCAAAATAA
SEQ ID Nr. 17: 
MKVVVVGCTHAGTAAVKTILNEHPDASVSVYERNDNVSFLSCGIALYVGGVVKDPAGLFYSSPEELASMGAKIN
MEHNVKNIDNENKVVVIENLKTGETFEESYDKLVMTTGSWPIIPPIDGINSENILLCKNYNQANEIIKESKNAKKIV
IVGGGYIAIELVEAFAESNKQVTLVARSDRILRKYLDAEFTSVLEHDLQERGVTLALNQTVEKFVANESGAVTAVK
TPVGEYEADLVILCVGFKPNTDLLKDKVEMLPNGAIVVDEYMRTSDEAIFAAGDSCAVHYNPTGGSAYIPLATN
AVRMGALVGKNIVSPTVKYRGTQATSGLYLFGFNIGSTGLTENSAPHFGVEVRSVVVEDNYRPEFMPTTEKVT
MKLVYEVGTNRIVGGQIMSKYDVTQSANTLSLCVQNKMTIEDLAYVDFFFQPHFDRPWNYLNILAQAAVEQER
KLAK
SEQ ID Nr. 18:

ATGAAAGTAGTAGTAGTAGGCTGTACACATGCAGGAACAGCGGCAGTTAAGACGATTTTAAATGAACATC
CAGATGCATCAGTATCAGTATATGAGCGTAATGACAATGTCTCATTTCTATCTTGTGGGATTGCGTTGTATG
TTGGTGGAGTTGTGAAAGATCCTGCAGGTTTGTTTTATTCAAGTCCAGAAGAACTTGCATCAATGGGCGCG
AAAATTAACATGGAACACAATGTGAAAAATATAGATAATGAGAATAAGGTCGTAGTAATTGAGAATTTAAA
AACAGGCGAAACATTTGAAGAAAGCTATGATAAGTTGGTAATGACAACTGGATCATGGCCAATTATTCCTC
CAATTGATGGAATCAATAGTGAAAATATTCTTTTGTGTAAAAACTATAACCAAGCAAATGAAATTATTAAAG
AATCAAAAAATGCTAAAAAGATTGTCATTGTTGGTGGTGGCTATATTGCGATTGAATTAGTTGAGGCATTT
GCAGAATCTAACAAGCAAGTGACGCTAGTTGCGCGTAGCGATCGTATTTTACGTAAATATTTAGATGCTGA
ATTCACTTCTGTTTTAGAGCATGATTTACAAGAAAGAGGCGTTACGCTAGCTTTAAACCAAACCGTCGAGAA
ATTTGTTGCCAATGAATCAGGTGCTGTGACAGCTGTGAAAACACCAGTTGGAGAATATGAGGCTGATTTAG
TTATTTTATGTGTTGGATTTAAACCAAATACTGATTTGTTGAAGGATAAAGTAGAGATGTTGCCAAATGGTG
CCATCGTAGTGGATGAATATATGAGAACAAGCGATGAAGCGATTTTTGCTGCTGGCGATAGTTGCGCGGTT
CATTATAATCCAACTGGAGGCTCTGCGTATATTCCGTTAGCTACAAATGCAGTTAGAATGGGAGCTTTAGTT
GGGAAAAATATTGTTTCTCCAACAGTTAAATATCGTGGCACGCAAGCAACTTCTGGTTTATATTTATTTGGT
TTTAATATAGGTTCAACCGGATTGACTGAAAATAGCGCTCCTCATTTTGGCGTAGAGGTTCGTTCAGTAGTT
GTAGAAGATAATTATCGTCCAGAGTTTATGCCGACAACAGAGAAAGTAACGATGAAATTAGTTTATGAAGT
AGGAACGAATCGGATTGTTGGAGGTCAAATCATGTCAAAATATGATGTGACACAATCTGCCAATACGTTAT

CTTTATGTGTTCAAAATAAAATGACGATTGAGGATTTGGCTTATGTAGATTTCTTCTTCCAACCTCACTTTGA
TCGTCCTTGGAACTATTTAAATATTTTAGCGCAAGCAGCTGTTGAGCAAGAGCGTAAACTAGCAAAATAA

Die Enzyme, die die Aminosäuresequenzen SEQ ID Nr. 11, SEQ ID Nr. 13, SEQ ID Nr. 15 oder SEQ ID Nr. 17 umfassen oder aus ihnen bestehen oder von den Nukleinsäuresequenzen SEQ ID Nr. 12, SEQ ID Nr. 14, SEQ ID Nr. 16 bzw. SEQ ID Nr. 18 kodiert werden, sind in der Lage, sowohl NADH als auch NADPH zu oxidieren.

Ein weiterer Aspekt der vorliegenden Erfindung betrifft eine Zusammensetzung enthaltend Glycolsäure und/oder Salze davon erhältlich nach einem erfindungsgemäßen Verfahren.

Mit den nachfolgenden Beispielen werden bevorzugte Ausführungsformen der Erfindung noch näher beschrieben.

### Materialien

Glycolaldehyd-Dimer, Ethylenglycol sowie IPTG (Isopropyl-β-D-thiogalactopyranosid) wurden von Sigma-Aldrich, Natrium-Glycolat wurde von AlfaAesar, Aceton, Kaliumdihydrogenphosphat, di-Kaliumhydrogenphosphat sowie Natriumdodecylsulfat (SDS) wurden von Carl Roth, NAD⁺, NADH-Dinatriumsalz, Zinkchlorid und Methanol wurden von PanReac AppliChem (ITW Reagents) und Triethanolamin wurde von Chem-Lab NV bezogen.

### Produktion der Enzyme & Herstellung der Lysate

### Allgemeines zur Expression von rekombinanten Enzymen in E. coli

Für die rekombinante Enzymproduktion in einem *Escherichia* coli-Stamm wurde zunächst das zu exprimierende Gen in einer PCR unter der Verwendung der genomischen DNA oder deren synthetisch an die Codon-Verwendung von *E. coli* angepasstes Äquivalent als Matrize zusammen mit spezifischen Oligonukleotiden, die zusätzlich Erkennungssequenzen für Restriktionsendonukleasen tragen, amplifiziert und aus dem Reaktionsgemisch isoliert. Nach dem Nukleinsäure-Verdau wurde das für das Target-Enzym kodierende Genfragment in das geschnittene Rückgrat des Expressionsvektors pQE70-Kan ligiert. Das Ligationsprodukt wurde in chemisch kompetente *E. coli*-Zellen Top10F transformiert und die entstandenen Kolonien wurden für die Plasmid-Isolierung und Restriktionsanalyse benutzt.

Das Ergebnis des Klonierungs-Schritts wurde mittels Restriktionsenzym-Verdau und DNA-Sequenzierung überprüft. Das resultierende Konstrukt trägt das Target-Gen unter dem IPTG-induzierbaren T5-Promotor.

Für die Überexpression des Enzymes in *E. coli* wurde das resultierende Expressionsplasmid in die kompetenten Expressionszellen RB791 transformiert. Nach 24 h Inkubation bei 37 °C wurden entstandene Kolonien für die Expressionstests in LB-Medium angeimpft.

Am nächsten Tag wurden damit Expressionskulturen mit einer optische Dichte OD₅₅₀ von 0,02 angeimpft und bei 37 °C geschüttelt, bis eine OD₅₅₀ von 0,3 erreicht wurde. Anschließend wurde die Temperatur auf 25 °C gesenkt und die Kulturen wurden beim Erreichen einer OD₅₅₀ von 0,5 mit 0,1 mM IPTG induziert. Nach 22 h wurden die Kulturen geerntet (vom Medium mittels Zentrifugation in Form eines Zellpellets abgetrennt) und auf die Expression des rekombinanten Enzymes mit Hilfe von SDS-Gelelektrophorese und einer Aktivitätsbestimmung (Verwendung in Use-Test oder optisch-enzymatischer Assay) analysiert.

### Herstellung von Zell-Lysaten mittels Sonifier-Aufschlusses

Zur Herstellung einer Zell-Suspension wurde das nach obigen Verfahren hergestellte Zellpellet in einem geeigneten Gefäß eingewogen und mit Puffer versetzt und unter Rührung gelöst. Der Massenanteil an Biomasse beträgt üblicherweise 20%, der Rest entfällt auf den Puffer.

Zum Zell-Aufschluss wurde ein Branson Sonifier 450 verwendet. Die Suspension wurde dreimal mit je 15 Ultraschall-Stößen (Einstellungen am Gerät: Timer = 15; Duty Cycle = 50; Output Control = 3 - 5) behandelt.

Das erhaltene Homogenat wurde 10 min lang bei 4 °C und 16000 rpm zentrifugiert (Eppendorf Zentrifuge 5417R), um die unlöslichen Zellfragmente abzutrennen und das Lysat zu erhalten.

**Tabelle 1. Spenderorganismen und Aufschlussbedingungen für die in den Beispielen verwendeten Enzyme.**

| **Enzymtyp (EC-Klasse)** | **katalysierte Reaktion(en)** | **Spenderorganismus** | **Literatur** |
|---|---|---|---|
| Alkoholdehydrog enase I (EC 1.1.1.1) | Ethylenglycol → Glycolaldehyd; | *Geobacillus stearothermophilus* | (NCBI Protein Database: WP_033015595.1); SEQ ID Nr. 1 |
| | Aceton → 2-Propanol | | |
| Alkoholdehydrog enase II (EC 1.1.1.1) | Ethylenglycol → Glycolaldehyd; | *Acetobacter pasteurianus* | (NCBI Protein Database: AEN03783.1); SEQ ID Nr. 3 |
| | Aceton → 2-Propanol | | |
| Alkoholdehydrog enase III (EC 1.1.1.1) | Ethylenglycol → Glycolaldehyd | *Achromobacter xylosoxidans* | (NCBI Protein Database: WP_006385522.1); SEQ ID Nr. 5 |
| Glycolaldehyd-Dehydrogenase (EC 1.2.1.21) | Glycolaldehyd → Glycolsäure | *Escherichia coli* | (Hidalgo et al., 1991) |
| NADH-Oxidase I (EC 1.6.3.4) | NADH → NAD⁺ | *Carnobacterium divergens* | SEQ ID Nr. 7 |
| NADH-Oxidase II (EC 1.6.3.4) | NADH → NAD⁺ | *Streptococcus mutans* | (Matsumoto et al., 1996); SEQ ID Nr. 9 |

### Analytische Methoden

### High Performance Liquid Chromatography (HPLC)

Zur Quantifizierung von Ethylenglycol, Glycolaldehyd und Glycolsäure wurde HPLC (High Performance Liquid Chromatography) verwendet. Die Detektion erfolgte mittels eines Brechungsindex-Detektors. Zur Messung wurde eine Phenomenex Rezex ROA-Organic Acid H+ (8%) Säule mit entsprechender Vorsäule verwendet und mit 1 mM Schwefelsäure isokratisch eluiert.

### Bestimmung von Enzym-Aktivitäten (optisch-enzymatischer Assay)

Enzym-Aktivitäten in den Lysaten wurden mit einem Shimadzu UV-1900 Spektrophotometer bestimmt. Dazu wurde die Bildung bzw. der Verbrauch von NAD(P)H bei einer Wellenlänge von 340 nm über die Änderung der Absorption verfolgt. Die Messungen wurden mit 0,2 mM Kofaktor (NAD(P)⁺oder NAD(P)H) durchgeführt. Dazu wurden 20 µl einer 10 mM Stock-Lösung des Kofaktors in einer Küvette (Greiner bioone Semi-Micro-Küvette aus Polystyrol) vorgelegt und der gewünschte pH-Wert wurde mit 100 mM TEA-HCl-Puffer eingestellt (870 µl). 10 µl Lysat (verdünnt oder unverdünnt) und 100 µl Substrat-Lösung wurden zur Küvette zugesetzt und die Messung unmittelbar danach gestartet. Die Messungen wurden standardmäßig bei 25 °C durchgeführt. Über den Extinktionskoeffizienten von NADH bzw. NADPH bei 340 nm (ε = 6220 L mol⁻¹ cm⁻¹) kann die Enzym-Aktivität des Lysats in U/ml (bezogen auf das Volumen des Lysats) oder U/g (bezogen auf die zur Herstellung eingesetzte Biomasse) bestimmt werden. 1 U steht dabei für 1 µmol Substratumsatz pro Minute (1 U = 1 µmol/min = 1,67·10⁻⁸ kat).

Mit den nachfolgenden Beispielen werden bevorzugte Varianten des erfindungsgemäßen Verfahrens noch näher beschrieben. Die in diesen Beispielen eingesetzten Lysate wurden nach den oben beschriebenen Verfahren hergestellt.

### Beispiel 1

### Oxidation von Ethylenglycol zu Glycolsäure - Kofaktor-Recycling mittels NADH-Oxidase

Die Reaktion wurde in einem Multifors Tisch-Bioreaktor (Infors AG) durchgeführt. Als Gefäß wurde ein Glasreaktor (Volumen 1,0 I) mit aufgesetztem Rührwerk, pH-Elektrode und O₂-Sensor verwendet. Die pH-Kontrolle erfolgte durch die Zudosierung von 5M NaOH.

Zu Beginn wurden 50 ml einer Ethylenglycol-Lösung (3,87 M), 173,6 ml deionisiertes Wasser sowie 7,5 ml eines 2M TEA-HCl-Puffers (pH 8) im Reaktor vorgelegt und unter Rühren auf 25 °C gebracht. Um Schaumbildung im Reaktor zu unterbinden bzw. zu reduzieren wurde ein Antischaummittel zugesetzt (Glanapon 2000; Bussetti und Co., GmbH, Österreich).

Zum Start der Reaktion wurden 24 ml Alkoholdehydrogenase II-Lysat, 5 kU NADH-Oxidase I, 5 kU Glycolaldehyd-Dehydrogenase-Lysat sowie 10 ml einer 6 mM NAD⁺-Lösung (finale Konzentration 0,2 mM) eingebracht. Zur Oxidation von NADH zu NAD⁺ mittels NADH-Oxidase wurde der Reaktionslösung konstant Luft wurde mit einem Fluss von 0,6 L/min über einen Sparger zugeführt.

Während des Betriebs wurden laufend Proben von der Reaktorlösung genommen, die folgendermaßen analysiert wurden: 50 µl des Ansatzes wurden zuerst mit 150 µl Reinstwasser verdünnt. Danach wurden 40 µl der verdünnten Lösung mit 160 µl Reinstwasser und 200 µl MeOH versetzt und bei 60 °C für 20 min inkubiert (1200 rpm, Eppendorf Thermomixer). Nach der Denaturierung wurde die Probe 10 min bei max. g zentrifugiert. 150 µl des klaren Überstands wurden in HPLC-Vials überführt und vermessen (HPLC, RI-Detektion).

Nach 17,5 h wurden 95% des Ethylenglycols (644 mM) zu Glycolsäure oxidiert. Nach Beendigung der Reaktion (42 h; 97% Umsatz) wurde der pH-Wert der Reaktorlösung auf pH 4 mit H₂SO₄ gestellt und der Reaktorinhalt für 1 h bei 60 °C gerührt, um die Enzyme zu deaktivieren. Die Enzyme wurden zuerst über einen Papierfilter im Büchner-Trichter abgetrennt und die erhaltene trübe Lösung wurde nochmals mittels Glasfritte (P4) filtriert, wodurch eine klare Lösung erhalten wurde.

### Beispiel 2

### Oxidation von Ethylenglycol zu Glycolsäure - Kofaktor-Recycling mittels Alkoholdehydrogenase und Aceton

In einem 2 ml Glas-Vial wurden folgende Komponenten vermischt: 100 µl eines 1000 mM TEA-HCl-Puffers (pH 9), 204 µl deionisiertes Wasser, 100 µl einer Ethylenglycol-Lösung (813 mM), 40 µl Alkoholdehydrogenase II-Lysat, 4 U Glycolaldehyd-Dehydrogenase-Lysat, 15 µl einer 10 mM NAD⁺-Lösung (finale Konzentration 0,3 mM) sowie 15 µl Aceton. Das Gesamtvolumen des Ansatzes betrug 500 µl.

Der Ansatz wurde 20 h bei 25 °C unter kontinuierlichem Schütteln (1200 rpm, Eppendorf Thermomixer) inkubiert. 4 h nach Start der Reaktion wurden 20 µl Aceton nachdosiert.

Zur Aufarbeitung wurden 50 µl des Ansatzes mit 150 µl Reinstwasser und 200 µl MeOH versetzt und bei 60 °C für 20 min inkubiert (1200 rpm, Eppendorf Thermomixer). Nach der Denaturierung wurde die Probe 10 min bei max. g zentrifugiert. 150 µl des klaren Überstands wurden in HPLC-Vials überführt und vermessen (HPLC, RI-Detektion).

Auf diese Weise konnten 50% des Ethylenglycols (163 mM) zu Glycolsäure oxidiert werden.

### Literatur

Sharad J. (2013). Glycolic acid peel therapy - a current review. Clinical, Cosmetic and Investigational Dermatology, 6, 281-288. https://doi.org/10.2147/CCID.S34029
Panova, A., Mersinger, L. J., Liu, Q., Foo, T., Roe, D. C., Spillan, W. L., Sigmund, A. E., Ben-Bassat, A., Wagner, L. W., O'Keefe, D., Wu, S., Petrillo, K., Payne, M. S., Breske, S. T., Gallagher, F. G., & DiCosimo, R. (2007). Chemoenzymatic Synthesis of Glycolic Acid. Advanced Synthesis & Catalysis, 349(8-9), 1462-1474. https://doi.org/10.1002/adsc.200700061
Ben-Bassat, A., Walls, A. M., Plummer, M. A., Sigmund, A. E., Spillan, W., & DiCosimo, R. (2008). Optimization of Biocatalyst Specific Activity for Glycolic Acid Production. Advanced Synthesis & Catalysis, 350(11-12), 1761-1769. https://doi.org/10.1002/adsc.200800228
Dapsens, P.Y., Mondelli, C., Kusema, B.T., Verel, R., & Pérez-Ramírez, J. (2014). A continuous process for glyoxal valorisation using tailored Lewis-acid zeolite catalysts. Green Chemistry, 16(3), 1176-1186. https://doi.org/10.1039/C3GC42353K
Tschirner, S., Weingart, E., Teevs, L., & Prüße, U. (2022). Oxidation of Monoethylene Glycol to Glycolic Acid with Gold-Based Catalyst and Glycolic Acid Isolation by Electrodialysis. Reactions, 3, 47-58. https://doi.org/10.3390/reactions3010004
Berger, A. (2016). Ethylenglycol, RD-05-01999 in Böckler, F., Dill, B., Eisenbrand, G., Faupel, F., Fugmann, B., Gamse, T., Matissek, R., Pohnert, G., Rühling, A., Schmidt, S., & Sprenger, G. (Ed.), RÖMPP [Online], Stuttgart, Georg Thieme Verlag. https://roempp.thieme.de/lexicon/RD-05-01999 (aufgerufen am 24.10.2023)
Wong, M. K., Lock, S. S. M., Chan, Y. H., Yeoh, S. J., & Tan, I. S. (2023). Towards sustainable production of bio-based ethylene glycol: Progress, perspective and challenges in catalytic conversion and purification. Chemical Engineering Journal, 468, 143699. https://doi.org/10.1016/j.cej.2023.143699
Tullo, A. H. (2017). Haldor Topsoe, Braskem investigate biobased ethylene glycol. Chemical and Engineering News, 95(46). Aufrufbar unter: https://cen.acs.org/articles/95/i46/Haldor-Topsoe-Braskem-investigate-biobased.html (aufgerufen am 10.10.2023)
Kataoka, M., Sasaki, M., Hidalgo, A.-R. G. D., Nakano, M., & Shimizu, S. (2001). Glycolic acid production using ethylene glycol-oxidizing microorganisms. Bioscience, Biotechnology, and Biochemistry, 65(10), 2265-2270. https://doi.org/10.1271/bbb.65.2265
Carniel, A., Santos, A. G., Chinelatto, L. S., Castro, A. M., & Coelho, M. A. Z. (2023). Biotransformation of ethylene glycol to glycolic acid by Yarrowia lipolytica: A route for poly(ethylene terephthalate) (PET) upcycling. Biotechnology Journal, 18, e2200521. https://doi.org/10.1002/biot.202200521
Zhang, H., Shi, L., Mao, X., Lin, J., & Wei, D. (2016). Enhancement of cell growth and glycolic acid production by overexpression of membrane-bound alcohol dehydrogenase in Gluconobacter oxydans DSM 2003. Journal of Biotechnology, 237, 18-24. https://doi.org/10.1016/j.jbiotec.2016.09.003
Hua, X., Du, G., & Xu, Y. (2019). Cost-practical of glycolic acid bioproduction by immobilized whole-cell catalysis accompanied with compressed oxygen supplied to enhance mass transfer. Bioresource Technology, 283, 326-331. https://doi.org/10.1016/j.biortech.2019.03.094
Boronat, A., Caballero, E., & Aguilar, J. (1983). Experimental Evolution of a Metabolic Pathway for Ethylene Glycol Utilization by Escherichia coli. Journal of Bacteriology, 153(1), 134-139. https://doi.org/10.1128/jb.153.1.134-139.1983
Pandit, A. V., Harrison, E., & Mahadevan, R. (2021). Engineering Escherichia coli for the utilization of ethylene glycol. Microbial Cell Factories, 20, 22. https://doi.org/10.1186/s12934-021-01509-2
Zhang, X., Zhang, B., Lin, J., & Wei, D. (2015). Oxidation of ethylene glycol to glycolaldehyde using a highly selective alcohol dehydrogenase from Gluconobacter oxydans. Journal of Molecular Catalysis B: Enzymatic, 112, 69-75. https://doi.org/10.1016/j.molcatb.2014.12.006
Gao, X., Ma, Z., Yang, L., & Ma, J. (2014). Enhanced Bioconversion of Ethylene Glycol to Glycolic Acid by a Newly Isolated Burkholderia sp. EG13. Applied Biochemistry and Biotechnology, 174, 1572-1580. https://doi.org/10.1007/s12010-014-1114-9
Yamada, M., & Isobe, K. (2015). A Novel Microbial Aldehyde Oxidase Applicable to Production of Useful Raw Materials, Glycolic Acid and Glyoxylic Acid, from Ethylene Glycol. Fermentation Technology, 4(1), 1000116. https://doi.org/10.4172/2167-7972.1000116
Yamada, M., Adachi, K., Ogawa, N., Kishino, S., Ogawa, J., Kataoka, M., Shimizu, S., & Isobe, K. (2015). A new aldehyde oxidase catalyzing the conversion of glycolaldehyde to glycolate from Burkholderia sp. AlU 129. Journal of Bioscience and Bioengineering, 119(4), 410-415. https://doi.org/10.1016/j.jbiosc.2014.09.005
Al-Rabiah, A. A., Boz, I., Akhmedov, V. M., Mostafa, M. M. M., & Bagabas, A. A. (2022). Highly Selective Gas-Phase Catalytic Hydrogenation of Acetone to Isopropyl Alcohol. Catalysts, 12(10), 1251. https://doi.org/10.3390/catal12101251
Altschul, S. F., Gish, W., Miller, W., Myers, E. W., & Lipman, D. J. (1990). Basic local alignment search tool. Journal of Molecular Biology, 215(3), 403-410. https://doi.org/10.1016/S0022-2836(05)80360-2
Henikoff, S., & Henikoff, J. G. (1992). Amino acid substitution matrices from protein blocks. Proceedings of the National Academy of Sciences of the United States of America, 89(22), 10915-10919. https://doi.org/10.1073/pnas.89.22.10915
Sambrook, J., Fritsch, E. R., & Maniatis, T. (1989). Molecular Cloning: A Laboratory Manual (2nd ed.). Cold Spring Harbor, NY: Cold Spring Harbor Laboratory Press.
Protein [Internet]. Bethesda (MD): National Library of Medicine (US), National Center for Biotechnology Information. Accession No. WP_033015595.1, alcohol dehydrogenase AdhP [Geobacillus stearothermophilus]; [aufgerufen am 10.10.2023]. Verfügbar unter: https://www.ncbi.nlm.nih.gov/protein/WP_033015595.1
Protein [Internet]. Bethesda (MD): National Library of Medicine (US), National Center for Biotechnology Information. Accession No. AEN03783.1, NAD+-dependent and Zn-containing alcohol dehydrogenase [Acetobacter pasteurianus NBRC 101655]; [aufgerufen am 10.10.2023]. Verfügbar unter: https://www.ncbi.nlm.nih.gov/protein/AEN03783.1
Protein [Internet]. Bethesda (MD): National Library of Medicine (US), National Center for Biotechnology Information. Accession No. WP_006385522.1, alcohol dehydrogenase AdhP [Achromobacter xylosoxidans]; [aufgerufen am 10.10.2023]. Verfügbar unter: https://www.ncbi.nlm.nih.gov/protein/WP_006385522.1
Hidalgo, E., Chen, Y.-M., Lin, E. C. C., & Aguilar, J. (1991). Molecular cloning and DNA sequencing of the Escherichia coli K-12 ald gene encoding aldehyde dehydrogenase. Journal of Bacteriology, 173(19), 6118-6123. https://doi.org/10.1128/jb.173.19.6118-6123.1991
Matsumoto, J., Higuchi, M., Shimada, M., Yamamoto, Y., & Kamio, Y. (1996). Molecular Cloning and Sequence Analysis of the Gene Encoding the H2O-forming NADH Oxidase from Streptococcus mutans. Bioscience, Biotechnology, and Biochemistry, 60(1), 39-43. https://doi.org/10.1271/bbb.60.39

## Patentansprüche

1. Verfahren zur Herstellung einer wässerigen Lösung enthaltend Glycolsäure, indem Ethylenglycol, welches in einer wässerigen Lösung vorliegt, durch Behandeln mit einer ersten NAD(P)-abhängigen Oxidoreduktase und NAD(P)⁺ *in vitro* zu Glycolaldehyd oxidiert und dieser mit einer weiteren NAD(P)-abhängigen Oxidoreduktase und NAD(P)⁺ *in vitro* zu Glycolsäure oxidiert und das durch diese Oxidationen entstandene reduzierte NAD(P)H mittels einer NAD(P)H-Oxidase oder einer Alkoholdehydrogenase wieder oxidiert und damit regeneriert wird.

2. Verfahren nach Anspruch 1, **dadurch gekennzeichnet, dass** die erste Oxidoreduktase eine Alkoholdehydrogenase und/oder die weitere Oxidoreduktase eine Aldehyd-Dehydrogenase ist.

3. Verfahren nach Anspruch 2, **dadurch gekennzeichnet, dass** die Aldehyd-Dehydrogenase eine Glycolaldehyd-Dehydrogenase ist.

4. Verfahren nach einem der Ansprüche 1 bis 3, **dadurch gekennzeichnet, dass** zur Oxidation des Kofaktors NAD(P)H eine Alkoholdehydrogenase unter Einsatz eines Ketons oder Aldehyds verwendet, wodurch *in vitro* NAD(P)⁺ und ein sekundärer oder ein primärer Alkohol gebildet werden.

5. Verfahren nach einem der Ansprüche 1 bis 3, **dadurch gekennzeichnet, dass** zur Oxidation des Kofaktors NAD(P)H eine NAD(P)H-Oxidase unter Einsatz von Sauerstoff verwendet wird, wodurch *in vitro* NAD(P)⁺ und Wasser gebildet werden.

6. Verfahren nach Anspruch 5, **dadurch gekennzeichnet, dass** NAD(P)H-Oxidase eine Aminosäuresequenz umfasst, welche ausgewählt ist aus der Gruppe bestehend aus:
i) einer Aminosäuresequenz, die eine Identität zu SEQ ID Nr. 7 oder SEQ ID Nr. 9 von mindestens 80% aufweist,
ii) einer Aminosäuresequenz, die durch eine Nukleinsäure kodiert wird, die eine Identität zu SEQ ID Nr. 8 oder SEQ ID Nr. 10 von mindestens 80% aufweist, und
iii) einer Aminosäuresequenz, die durch eine Nukleinsäure kodiert wird, die unter stringenten Bedingungen an ein Nukleinsäuremolekül mit der Nukleinsäuresequenz SEQ ID Nr. 8 oder SEQ ID Nr. 10 bindet.

7. Verfahren nach einem der Ansprüche 2 oder 3, **dadurch gekennzeichnet, dass** die Alkoholdehydrogenase sowie die Glycolaldehyd-Dehydrogenase als Lysat der entsprechenden, sie bildenden Zellen vorliegen.

8. Verfahren nach einem der Ansprüche 1 bis 7, **dadurch gekennzeichnet, dass** die NAD(P)-abhängige Oxidoreduktase zur Oxidation von Ethylenglycol zu Glycolaldehyd eine Aminosäuresequenz umfasst, welche ausgewählt ist aus der Gruppe bestehend aus:
i) einer Aminosäuresequenz, die eine Identität zu SEQ ID Nr. 1, 3 oder 5 von mindestens 80% aufweist,
ii) einer Aminosäuresequenz, die durch eine Nukleinsäure kodiert wird, die eine Identität zu SEQ ID Nr. 2, 4 oder 6 von mindestens 80% aufweist, und
iii) einer Aminosäuresequenz, die durch eine Nukleinsäure kodiert wird, die unter stringenten Bedingungen an ein Nukleinsäuremolekül mit der Nukleinsäuresequenz SEQ ID Nr. 2, 4 oder 6 bindet.

9. Verwendung einer NAD(P)-abhängigen Oxidoreduktase zur Oxidation von Ethylenglycol zu Glycolaldehyd, wobei die NAD(P)-abhängige Alkoholdehydrogenase eine Aminosäuresequenz umfasst, welche ausgewählt ist aus der Gruppe bestehend aus:
i) einer Aminosäuresequenz, die eine Identität zu SEQ ID Nr. 1, 3 oder 5 von mindestens 80% aufweist,
ii) einer Aminosäuresequenz, die durch eine Nukleinsäure kodiert wird, die eine Identität zu SEQ ID Nr. 2, 4 oder 6 von mindestens 80% aufweist, und
iii) einer Aminosäuresequenz, die durch eine Nukleinsäure kodiert wird, die unter stringenten Bedingungen an ein Nukleinsäuremolekül mit der Nukleinsäuresequenz SEQ ID Nr. 2, 4 oder 6 bindet.

10. Zusammensetzung enthaltend Glycolsäure und/oder Salze davon erhältlich nach einem Verfahren nach einem der Ansprüche 1 bis 8.
